# EUROPEAN PATENT APPLICATION

(11) **EP 4 748 917 A1**
(43) Date of publication of application: **27.05.2026**
(21) Application number: 24215230.4
(22) Date of filing: 25.11.2024
(51) Int. Cl.: C12C 7/28, C12C 11/00, C12C 11/02, C12C 11/06, C12C 12/00, C12C 12/04, C12N 1/18

(54) **YEAST STRAIN FOR LOW-ALCOHOLIC BEER**

(71) Applicant: AB Mauri (UK) Ltd, London W1K 4QY (GB)
(72) Inventor: SOUFFRIAU, Ben, London, W1K 4QY (GB); CHEUNG, Caleb, London, W1K 4QY (GB)
(74) Representative: V.O.

(57) **Abstract**

The invention relates to a yeast cell that is capable of fermenting an aqueous fermentation medium comprising, per 100 mL, at least 0.8 g of a total of maltose and maltotriose, thereby producing alcohol in a concentration of 0.5 vol.% or less, based on the volume of the aqueous fermentation medium, , and that has an ability to decrease the pH of the aqueous fermentation medium by 0.7 pH units or more and/or that is capable of fermenting an aqueous fermentation medium comprising, per 100 mL, at least 0.8 g of a total of maltose and maltotriose and a total of glucose, fructose and sucrose of at most 0.8 g, thereby producing alcohol in a concentration of 0.5 vol.% or less, based on the volume of the aqueous fermentation mediumand that has an ability to convert at least 15 wt.% of said total of glucose, fructose and sucrose in a time of 6 h or less and/or that has an ability to convert at least 80 wt.% of said total of glucose, fructose and sucrose in a time of 16 h or less.

## Description

The invention relates to a yeast cell that is capable of fermenting an aqueous fermentation medium comprising, per 100 mL, at least 0.8 g of a total of maltose and maltotriose, thereby producing ethanol ("alcohol") in a concentration of 0.5 vol.% or less.

Beer is a beverage obtained from the fermentation of wort. During the fermentation of wort, a yeast converts sugar present in the wort into ethanol and carbon dioxide gas, giving the beer its alcohol and carbonation. Besides converting sugar, the yeast also typically converts or produces other flavor-imparting molecules, which provides the beer with a characteristic fermented beer taste.

Low alcohol or alcohol-free beer is a variant of beer wherein the content of alcohol has been reduced. The amount of alcohol that may be present for a beer to be considered "low alcohol" dependents on local regulations, but is usually below 1.2 vol.%. The demand for low alcohol or alcohol-free beer is rising, due to the negative health impact of alcohol and incompatibility of alcohol with certain activities or situations, e.g. driving, being pregnant, or use of certain medicines.

There are several methods known in the art to prepare a low alcohol or alcohol-free beer. One method is known as "cold contact fermentation" or "cold contact process". In such a method, wort is fermented at a relatively low temperature, wherein the yeast is not capable of converting fermentable sugars into alcohol. Thereby, a beer is obtained that contains little to no alcohol.

Low alcohol beers obtained through cold contacting are however known to suffer from worty off-taste. This is due to the presence of low amounts of aldehydes, in particular those derived from Strecker degradation. Efforts to remove these aldehydes from the low alcohol beer are known. However, removal requires additional processing steps and risks simultaneously removing other flavor components from the low alcohol beer, which is also undesirable.

It is furthermore observed that the pH of a cold contact beer (after fermentation) is usually relatively high, i.e. around 4.5 or more. Such a pH allows spoilage by microbes and furthermore lacks refreshing taste. Typically therefore, the addition of substantial amounts of acidulants to the wort and/or final beer is required to lower the pH to below around 4.2 or less, to obtain a beer that has acceptable microbial stability and refreshing taste.

Accordingly, there is a need for a low-alcohol beer wherein one or more of the above-mentioned problems has been overcome.

The inventors surprisingly found a yeast cell that is capable of fermenting an aqueous fermentation medium thereby producing alcohol in a concentration of 0.5 vol.% or less and that has an ability to decrease the pH of the aqueous fermentation medium by 0.7 pH units or more. Such a property of a yeast is advantageous in particular for preparing a low-alcoholic beer, because it allows the production of a fermented beverage that has not only low alcohol content (preferably below 1.0 vol.%), but also has a relatively low pH, without requiring to use substantial amounts of acidulants. This is advantageous, because the use of acidulants is associated with several disadvantages, including lower efficiency of trub sedimentation, potentially leading to poor clarity of the fermented beverage. The yeast cell further advantageously converts wort sugars selected from glucose, fructose and sucrose, into ethanol and carbon dioxide relatively fast. The fermented beverage further advantageously has a refreshing taste and no worty off-flavour in particular low content of diacetyl compounds, 4-vinyl guaiacol and Strecker aldehydes, in particular Strecker aldehydes selected from 2-methylpropanal, 2-methylbutanal, 3-methylbutanal, hexanal, 2-furfural and methional.

The invention therefore relates to a yeast cell that is capable of fermenting an aqueous fermentation medium comprising per 100 mL, at least 0.8 g of a total of maltose and maltotriose and a total of glucose, fructose and sucrose of at most 0.8 g, thereby producing alcohol in a concentration of 0.5 vol.% or less, based on the volume of the aqueous fermentation medium, and that has an ability to decrease the pH of the aqueous fermentation medium by 0.7 pH units or more.

The invention further relates to a yeast cell that is capable of fermenting an aqueous fermentation medium comprising, per 100 mL aqueous fermentation medium, at least 0.8 g of a total of maltose and maltotriose and a total of glucose, fructose and sucrose of at most 0.8 g, thereby producing alcohol in a concentration of 0.5 vol.% or less, based on the volume of the aqueous fermentation medium, preferably 0.2 vol.% alcohol or less, and that has an ability to convert at least 15 wt.% of said total of glucose, fructose and sucrose in a time of 8 h or less and/or that has an ability to convert at least 80 wt.% of said total of glucose, fructose and sucrose in a time of 16 h or less.

The invention further relates to a method for producing a fermented beverage, comprising
a) providing an aqueous fermentation medium comprising wort and a plurality of yeast cells yeast preparation according to the invention,
b) optionally reducing the pH of said aqueous fermentation medium with 0.4 pH units or more;
c) culturing the aqueous fermentation medium under conditions allowing the yeast to convert one or more of 2-methylpropanal, 2-methylbutanal, 3-methylbutanal, methional, E-2-nonenal, 4-vinyl guaiacol, hexanal and optionally diacetyl, preferably methional, present in the wort to a content below the flavor threshold and/or to convert one or more sucrose, fructose and glucose, thereby obtaining, optionally after dilution, an aqueous fermentation medium comprising at most 0.5 vol.% of alcohol, preferably at most 0.2 vol.% of alcohol, based on the total volume of the aqueous fermentation medium; and
d) optionally isolating a fermented beverage from said aqueous fermentation medium.

### Figures

Figure 1: CO₂ production of a fermentation using yeast cells as deposited with the National Measurement Institute (Victoria, Australia) under accession number V24/019646.
Figure 2: Production of alcohol in a fermentation sing yeast cells as deposited with the National Measurement Institute (Victoria, Australia) under accession number V24/019646.
Figure 3: Perceived intensities of worth/grains, fruity, hop, acidity, sweetness and body flavours of a beer fermented with V24/019646 compared to a beer fermented with Lallemand LoNa, or Fermentis SafAle LA01.

### Detailed description

The term "or" as used herein means "and/or" unless specified otherwise.

The term "a" or "an" as used herein means "at least one" unless specified otherwise.

The term "essential(ly)" is generally used herein to indicate that it has the general character or function of that which is specified. When referring to a quantifiable feature, this term is in particular used to indicate that it is more than 90 %, more in particular more than 95 %, even more in particular more than 98 % of the maximum that feature. The term `essentially free' is generally used herein to indicate that a substance is not present (below the detection limit achievable with analytical technology as available on the effective filing date) or present in such a low amount that it does not significantly affect the property of the product that is essentially free of said substance or that it is present in such a low amount (trace) that it does not need to be labelled on the packaged product that is essentially free of the substance. In practice, in quantitative terms, a product is usually considered essentially free of a substance, if the content of the substance is 0 - 0.1 wt.%, in particular 0 - 0.01 wt.%, more in particular 0 - 0.005 wt.%, based on total weight of the product in which it is present.

The term "about" in relation to a value generally includes a range around that value as will be understood by the skilled person. In particular, the range is from at least 10 % below to at least 10 % above the value, more specifically from 5 % below to 5 % above the value.

When referring to a "noun" (*e.g.* a compound, an additive *etc*.) in singular, the plural is meant to be included, unless specified otherwise.

The term "yeast" is generally known in the art to refer to a eukaryotic, single-celled microorganism, which is part of the fungus kingdom. In the context of the present invention, when referring to `a yeast cell', a living yeast cell is meant, unless otherwise specified. As used herein, a yeast cell is typically isolated from its natural environment.

In the context of this application, when using the term "parts per billion (ppb)", ppb by weight is indicated, unless otherwise specified.

### Yeast cell

Yeasts are frequently utilized as fermenting microorganisms in the preparation of many food and beverage products to convert fermentable sugars into ethanol and carbon dioxide. During the fermentation process other molecules are produced that affect the characteristics of the beer.

As mentioned herein above, the inventors surprisingly found a yeast cell that is capable of fermenting an aqueous fermentation medium thereby producing alcohol in a concentration of 0.5 vol.% or less and that has an ability to decrease the pH of the aqueous fermentation medium by 0.7 pH units or more. Such a yeast cell is advantageous in the production of low-alcoholic beverages with good antimicrobial properties and refreshing taste.

It is believed that the yeast according to the invention is not capable of converting maltose and maltotriose into ethanol, whilst producing a beer-like flavor profile and reducing the pH of the aqueous fermentation medium with at least 0.7 pH units or more.

Accordingly, the invention relates to a yeast cell that is capable of fermenting an aqueous fermentation medium comprising, per 100 mL aqueous fermentation medium, at least 0.8 g of a total of maltose and maltotriose and a total of glucose, fructose and sucrose of at most 0.8 g, thereby producing alcohol in a concentration of 0.5 vol.% or less, based on the volume of the aqueous fermentation medium, preferably 0.2 vol.% alcohol or less, and that has an ability to decrease the pH of the aqueous fermentation medium by 0.7 pH units or more.

Said aqueous fermentation medium comprises, per 100 mL, at least 0.8 g of a total of maltose and maltotriose, preferably at least 0.9 g, more preferably at least 1.0 g, more preferably at least 1.2 g, more preferably at least 1.5 g, even more preferably at least 2 g, even more preferably at least 2.5 g, most preferably at least 3 g.

Preferably, the w/w ratio between maltose and maltotriose is between about 5:1 and about 2:1, more preferably between about 4:1 and 3:1.

Said aqueous fermentation medium preferably comprises, per 100 mL, at least 0.8 g of maltose, preferably at least 0.9 g, more preferably at least 1 g of maltose, more preferably at least 1.5 g of maltose, even more preferably at least 2 g of maltose, in particular at least 2.5 g of maltose.

Said aqueous fermentation medium preferably comprises, per 100 mL, at least 0.2 g of maltotriose, more preferably at least 0.3 g, even more preferably at least 0.4 g, even more preferably at least 0.5 g.

Such contents of maltose and maltotriose are commonly found in wort, in particular at wort gravities commonly used to produce (low alcohol) beer.

Usually, said aqueous fermentation medium comprises further wort sugars, preferably one or more of fructose, glucose and sucrose. Typically, the total of sucrose, fructose and glucose is, per 100 mL, at most 0.8 g, preferably at most 0.7 g, even more preferably at most 0.5 g, most preferably at most 0.3 g. Preferably, the total of glucose, fructose and sucrose is at least 0.1 g, more preferably at least 0.2 g, even more preferably at least 0.5 g, per 100 mL aqueous fermentation medium. Preferably, said aqueous fermentation medium comprises, per 100 mL, between about 0 g and about 0.8 g of a total of fructose, glucose and sucrose, more preferably between about 0.1 g and about 0.7 g of fructose, glucose and sucrose, even more preferably between about 0.2 g and about 0.5 g of a total of glucose, fructose and sucrose.

Typically, said aqueous fermentation medium comprises non-fermentable sugars, preferably dextrins. Non-fermentable sugars, as used herein, refers to sugars that are not substantially fermentable by fermenting yeasts for producing alcohol, in particular for producing alcohol from wort. Typically non-fermentable sugars are relatively large sugars, e.g. comprising five sugar residues or more, such as ten sugar residues or more. Usually, said aqueous fermentation medium comprises, per 100 mL, at least 0.1 g of non-fermentable sugars, preferably dextrins, more preferably at least 0.5 g of non-fermentable sugars, preferably dextrins, even more preferably at least 0.9 g of non-fermentable sugars, preferably dextrins, in particular at least 1.2 g of non-fermentable sugars, preferably dextrins. Preferably, said aqueous fermentation medium comprises, per 100 mL, between about 0.1 g and about 3 g of non-fermentable sugars, preferably dextrins, more preferably between about 0.5 g and about 2 g of non-fermentable sugars, preferably dextrins.

The content of sugars is determinable using any suitable method known in the art, such as HPLC. Preferably, the sugar content is determinable using the method described in Example 1.

Preferably, said aqueous fermentation medium comprises, consists of or consists essentially of wort and a plurality of yeast cells. Preferably, said aqueous medium comprises, consists of or consists essentially of wort having a gravity of at most 5.7 °P. More preferably, said aqueous fermentation medium comprises, consists of or consists essentially of wort and a plurality of yeast cells in an amount of about 3.5 · 10⁶, wherein said aqueous fermentation medium comprises, per 100 mL, at least 0.8 g of a total of maltose and maltotriose and at most 0.8 g of a combination of sucrose, glucose and fructose, preferably wherein said aqueous fermentation medium comprises 2.4 g maltose, 0.58 g glucose, 0.70 g maltotriose, 0.09 g sucrose, 0.1 g of fructose and 0.9 g of dextrins, per 100 mL aqueous fermentation medium.

The content of alcohol produced with a yeast according to the invention is 0.5 vol.% or less, preferably 0.4 vol.% or less, more preferably 0.3 vol.% or less, even more preferably 0.2 vol.% or less, in particular 0.2 vol.% or less, based on the volume of the aqueous fermentation medium.

The yeast cell according to the invention has an ability to decrease the pH of an aqueous fermentation medium by 0.4 pH units or more, preferably 0.5 pH units or more, even more preferably 0.6 pH units or more, even more preferably 0.7 pH units or more, even more preferably 0.8 pH units or more, even more preferably 0.9 pH units or more, most preferably 1.0 pH units or more, without requiring the addition of an acidulant. The pH is determinable using standard methods known in the art for determining pH, such as by using a calibrated pH meter.

Said ability to decrease the pH is preferably determinable with a method comprising the steps of:
- providing an aqueous fermentation medium consisting essentially of wort and a plurality of yeast cells in a concentration of 3.5 · 10⁶ cells/ml, said fermentation medium comprising 54.6 g/L light malt extract, of which 2.4 g maltose, 0.58 g glucose, 0.70 g maltotriose, 0.09 g sucrose, 0.1 g of fructose and 0.9 g of dextrins, per 100 mL aqueous fermentation medium,;
- setting the pH of said fermentation medium to 4.75;
- culturing the fermentation medium at a temperature of 20 °C for a time of 72 h in a sealed container configured to prevent oxygen from entering the container;
- determining the pH of the cultured fermentation medium, wherein the yeast cell has an ability to decrease the pH of the aqueous fermentation medium or more if the pH of the cultured fermentation medium is 0.7 pH units lower than the pH of the aqueous fermentation medium prior to culturing.

Said aqueous fermentation medium preferably consists essentially of wort and a plurality of yeast cells according to the invention.

Preferably, said method for determining the pH is performed at laboratory scale, preferably at most 100 L, more preferably at most 80 L, more preferably at most 50 L. Preferably, at a laboratory scale, an amount of between about 0.25 L and about 100 L of aqueous fermentation medium is provided, more preferably between about 20 L and about 80 L. Alternatively, said method is performed at industrial scale, preferably using at least 100 L of aqueous fermentation medium, more preferably at least 300 L, even more preferably at least 500 L, even more preferably at least 700 L, most preferably at least 1000 L. Typically, between about 500 L and about 400000 L of aqueous fermentation medium is provided, more preferably between about 500 L and about 10000 L.

Said wort is obtainable by a process known per se. Preferably, said wort is obtainable from germinated barley. Said germinated barley is preferable contacted with hot water to extract sugars therefrom, thereby obtaining sweet wort. Preferably, hops or hops-derived products such as hop oils or hop bitters are added to the sweet wort to obtain the wort suitable for fermentation.

Preferably, said wort is obtainable by a process comprising the steps of:
- contacting crushed, germinated barley grains with water at a temperature of 63-72 °C for approximately 0.3-1.5 h, followed by increasing the temperature to 78 °C;
- separating the aqueous phase from the crushed germinated barley grains to obtain sweet wort;
- boiling the sweet wort to obtain boiled sweet wort; and
- adding hops or hop-derived products to the boiled sweet wort to obtain the wort.

It was further surprisingly found that the yeast cell according to the invention is capable of converting monosaccharidic sugars and sucrose relatively fast, at least substantially faster compared to commercially available strains for producing fermented beverage with low alcohol content, preferably below 0.5 vol.%. Having a relatively fast fermentation is advantageous for economical, hygiene and efficiency reasons. For example, having a relatively fast fermentation helps to prevent microbial contaminations.

The invention therefore further relates to a yeast cell that is capable of fermenting an aqueous fermentation medium comprising, per 100 mL, at least 0.8 g of a total of maltose and maltotriose and a total of glucose, fructose and sucrose of at most 0.8 g, thereby producing alcohol in a concentration of 0.5 vol.% or less, based on the volume of the aqueous fermentation medium, preferably 0.2 vol.% alcohol or less, and that has an ability to convert at least 15 wt.% of said total of glucose, fructose and sucrose in a time of 8 h or less, preferably 7 h or less, even more preferably 6 h or less, most preferably 5 h or less, and/or that has an ability to convert at least 80 wt.%, preferably at least 85 wt.%, more preferably at least 90 wt.%, even more preferably at least 92 wt.%, even more preferably at least 95 wt.%, even more preferably at least 99 wt.% of said total of glucose, fructose and sucrose in a time of 16 h or less, more preferably 15 h or less, even more preferably 14 h or less, even more preferably 13 h or less, even more preferably 12 h or less, even more preferably 11 h or less, most preferably 10 h or less.

Said aqueous fermentation medium and alcohol content are as defined herein above.

Said ability of converting sucrose, fructose and glucose into ethanol in a defined amount of time is determinable by measuring the CO₂ pressure over time. CO₂ is released during the conversion of sugars by the yeast. Hence, the amount of CO₂ that is developed over time is an indication of the amount of sugars that have been converted by the yeast.

Accordingly, said ability is preferably determinable using a method comprising the steps of:
- providing a fermentation medium consisting essentially of wort and a plurality of yeast cells in a concentration of 3.5 · 10⁶ cells/ml, said fermentation medium comprising at least 0.8 g of a total of maltose and maltotriose and a total of glucose, fructose and sucrose of at most 0.8 g, more preferably comprising 2.4 g maltose, 0.58 g glucose, 0.70 g maltotriose, 0.09 g sucrose, 0.1 g of fructose and 0.9 g of dextrins, per 100 mL aqueous fermentation medium;
- preferably setting the pH of said fermentation medium to 4.75;
- culturing the fermentation medium at a temperature of 20 °C for a time of 72 h in a sealed container configured to prevent oxygen from entering the container;
- measuring the CO₂ pressure over time and determining the amount of said total of glucose, sucrose and fructose based on the CO₂ pressure.

Said CO₂ pressure is preferably determined using an automated CO₂ release valve and monitoring system.

The ability to convert at least 15 wt.% of said total of glucose, fructose and sucrose in a time of 8 h or less and/or at least 80 wt.% in a time of 16 h or less may be determined by monitoring the total of CO₂ formed during the fermentation and estimate the time at which 15% or 80% of the total amount of CO₂ was formed.

Good results have in particular be achieved with a yeast cell as deposited with the National Measurement Institute (Victoria, Australia) under accession number V24/019646 or a cell of a derivative strain thereof. V24/019646 has been deposited on November 6, 2024 with the National Measurement Institute (1/153 Bertie Street, Port Melbourne, Victoria, Australia 3207) under accession number V24/019646 on the same terms as laid down in the Budapest Treaty. The deposit was made by AB Mauri Technology & Development Pty. Ltd., 1 Richardson Place, North Ryde NSW, 2113, Australia. The depositor has authorised the applicant to refer to the deposited material in the application and has given his unreserved and irrevocable consent to the deposited material being made available to the public in accordance with Rule 31(1)(d) of the European Patent Convention (EPC). A statement of authorisation and consent is being filed with this application.

Accordingly, the invention further relates to a yeast cell as deposited with the National Measurement Institute (Victoria, Australia) under accession number V24/019646 or a cell of a derivative strain thereof.

A "derivative strain", as used herein, is a strain derived from a yeast cell disclosed herein (such as a yeast cell having an activity as defined herein or a yeast cell as deposited under accession number V24/019646), including through hybridization, mutagenesis, recombinant DNA technology, mating, cell fusion, or cytoduction between strains. A derivative strain can be a strain found in nature (a natural strain), yet isolated from its natural environment. Advantageously, the derivative strain is not found in nature (a non-natural strain). Advantageously, the derivative strain is a progeny strain of a parent strain (i.e. a strain from which it is a progeny), which progeny strain typically is the product of a mating between said parent strain and another parent strain or the progeny strain is a descendent of that mating product.

In an embodiment, the derivative strain is a hybrid strain that is producible or has been produced by a method comprising using a strain (such as a yeast cell having an activity as defined herein or a yeast cell as deposited under accession number V24/019646) as a parent strain for hybridisation.

In a preferred embodiment, the progeny strain can be obtained using a hybridization method comprising hybridization of a parent strain (such as a yeast cell having an activity as defined herein or a yeast cell as deposited under accession number V24/019646) and at least one step of screening or selection of the hybrid obtained, which selection is based on one or more defining characteristics of said parent strain. For instance, a defining characteristic can be based on taxonomic classification. Preferably, a defining characteristic is an ability of decreasing the pH of the aqueous fermentation medium by 0.7 pH units or more. Said ability being determinable using the method defined in claim 3. Alternatively or additionally, a defining characteristic is an ability to convert at least 15 wt.% of said total of glucose, fructose and sucrose in a time of 8 h or less, preferably 6 h or less and/or that has an ability to convert at least 80 wt.% of said total of glucose, fructose and sucrose in a time of 16 h or less, preferably 12 h or less.

In a particularly preferred embodiment, the derivative strain is obtained or obtainable by a method comprising,
(a) providing:
   (i) a first strain (such as a yeast cell having an activity as defined herein or a yeast cell as deposited under accession number V24/019646); and
   (ii) a second strain, wherein the second strain is in the same clade as the first strain;
(b) inducing mating (e.g. sporulation or conjugation) of the first and the second strain;
(c) screening or selecting for a derivative strain.

In a particularly preferred embodiment, the derivative strain of a spore-forming strain is obtained or obtainable by a method comprising,
(a) providing:
   (i) a first strain such as a yeast cell having an activity as defined herein or a yeast cell as deposited under accession number V24/019646; and
   (ii) one or more additional strains that are in the same clade as the first strain;
(b) inducing sporulation of the first strain and the one or more additional strains to produce spores;
(c) mixing the spores of step (b) to allow for hybridization of the spores; and
(d) screening or selecting for the derivative strain.

In a further preferred embodiment, the derivative strain is an evolved strain, in particular a strain obtained or obtainable by an evolution induced by applying selection pressure to the parent strain or a derivative thereof).

In a further preferred embodiment, the derivative strain is a mutant strain of the parent strain or a derivative thereof. A mutant can be obtained by contacting the derivative with a mutagen. Examples of mutagens are ethyl methanesulfonate (EMS), ultraviolet light (UV), X-rays, methylmethane sulphonate (MMS), nitrous acid, nitrosoguanidine (NNG), acridine mustard, 2-methoxy-6-chloro-9[3- (ethyl-2-chloroethyl)aminopropylamino]acridine ·2 (ICR-170), and nitrogen mustard.

In a further preferred embodiment, the derivative strain is a genetically modified strain. In particular, the genetically modified strain has a modification made using gene editing (also called genome editing). The gene editing can in particular comprise a modification to suppress expression of a gene, enhance expression of a gene, introduce one or more genomic nucleic acids, delete one or more genomic nucleic acids, introduce a gene, or delete a gene.

A yeast cell according to the invention is preferably a cell of the genus *Saccharomyces.*

Preferably said yeast cell is a cell selected from the group consisting of *Saccharomyces arboricolus, Saccharomyces bayanus, Saccharomyces bulderi, Saccharomyces cariocanus, Saccharomyces cariocus, Saccharomyces cerevisiae, Saccharomyces chevalieri, Saccharomyces dairenensis, Saccharomyces ellipsoideus, Saccharomyces eubayanus, Saccharomyces exiguous, Saccharomyces florentinus, Saccharomyces fragilis, Saccharomyces kudriavzevii, Saccharomyces ludwigii, Saccharomyces martiniae, Saccharomyces mikatae, Saccharomyces norbensis, Saccharomyces paradoxus, Saccharomyces pastorianus, Saccharomyces spenderorum, Saccharomyces turicensis, Saccharomyces unisporus, Saccharomyces uvarum* and *Saccharomyces zonatus,* more preferably *Saccharomyces cerevisiae.*

A yeast cell according to the invention may be formulated in any suitable way. Accordingly, the invention further relates to a yeast preparation, comprising a yeast cell according to the invention, wherein the preparation is formulated as a dry preparation, a liquid preparation, a cream preparation, a crumble preparation or a compressed preparation.

A dry preparation, liquid preparation, cream preparation, crumble preparation or a compressed yeast preparation may be obtained using any suitable method known in the art.

### Method

The invention further relates to a method for producing a fermented beverage, comprising
a) providing an aqueous fermentation medium comprising wort and a plurality of yeast cells or a yeast preparation according to the invention,
b) optionally reducing the pH of said aqueous fermentation medium with 0.4 pH units or more;
c) culturing the aqueous fermentation medium under conditions allowing the yeast to convert one or more of 2-methylpropanal, 2-methylbutanal, 3-methylbutanal, methional, E-2-nonenal, 4-vinyl guaiacol, diacetyl and hexanal, preferably hexanal, present in the wort to a content below the flavor threshold and/or to convert one or more sucrose, fructose and glucose, thereby obtaining, optionally after dilution, an aqueous fermentation medium comprising at most 0.5 vol.% of alcohol, preferably at most 0.2 vol.% of alcohol, based on the total volume of the aqueous fermentation medium; and
d) optionally isolating a fermented beverage from said aqueous fermentation medium.

The aqueous fermentation medium comprises wort. As is known, wort is a natural product derived from malted cereal grains. Malted cereal grains are grains that have been allowed to germinate by soaking the grains in water, to allow conversion of starch into small sugars (i.e. sugars containing four or less monosaccharides).

In principle, any type of wort may be employed in a method according to the invention. Preferably, said wort is derived from malted barley, malted maize, malted oat, malted millet, malted triticale, malted fonio, malted wheat, malted rye or malted sorghum, more preferably malted barley and/or malted wheat grains.

Typically, wort is prepared by mashing malted cereal grains to convert starch into fermentable sugars. Subsequently, the wort is usually sterilized, e.g. boiled, to obtain a sterilized wort. Usually, hops, hop pellets, hop-derived products such as hop oils or hop extracts and optionally other flavor molecules are added to the wort prior to sterilization. Examples of hop-derived products include myrcene, humulene, humulone, caryophyllene and farnesene and derivatives thereof.

Preferably, said wort is obtainable by a process comprising the steps of:
- contacting crushed, germinated cereal grains with water at elevated temperature thereby allowing conversion of starch into small sugars;
- separating the aqueous phase from the crushed germinated cereal grains to obtain sweet wort;
- sterilizing, preferably boiling, the sweet wort to obtain sterilized sweet wort; and
- adding hops or hop-derived products to the sterilized sweet wort to obtain the wort.

Optionally the liquid fraction of said wort may be removed, and the solid fraction may be reconstituted in water prior to use in a method according to the invention, or a method for determining an ability according of a yeast according to the invention.

Said crushed germinated cereal grains are preferably obtained by keeping cereal grains under conditions allowing them to germinate. Typically, said cereal grains are kept in a germination chamber for at least 24 h, more preferably at least 48 h, such as at least 72 h. After said cereal grains have germinated (malted), said germinated grains are usually dried ("kilned') at elevated temperature.

The temperature at which said crushed, germinated cereal grains are contacted with water is a temperature at which starch-converting enzymes, preferably amylase, are active. Preferably said elevated temperature is at least 50 °C, more preferably at least 55 °C, even more preferably at least 60 °C. Preferably, said elevated temperature is at most 80 °C, more preferably at most 75 °C, even more preferably at most 70 °C. Preferably said elevated temperature is between about 50 °C and about 80 °C, more preferably between about 55 °C and about 75 °C, even more preferably between about 60 °C and about 70 °C.

Usually, said germinated barley grains are contacted for a time of at least 15 minutes, more preferably at least 30 minutes, more preferably at least 45 minutes, in particular at least 60 minutes. Preferably, said germinated barley grains are contacted for a time between about 15 minutes and 360 minutes, more preferably between about 30 minutes and about 120 minutes.

Subsequently, said aqueous phase containing sugars is separated from said crushed germinated cereal grains to obtain sweet wort. This may be achieved using any suitable means in the art. For example, the aqueous mixture may be separated in a lauter tun.

Said sweet wort is sterilized, preferably by heating said sweet wort to a temperature of at least 70 °C, more preferably at least 75 °C, even more preferably at least 80 °C, in particular at least 85 °C, more in particular at least 90 °C, most preferably at least 95 °C, such as at least 100 °C, thereby removing microbes from the sweet wort.

Preferably, hop extract is added to said sterilized sweet wort to impart a beer-like taste to the wort.

Usually said aqueous fermentation medium comprises sugars, in particular one or more sugars selected from maltose, maltotriose, fructose, sucrose and glucose. Preferably, said aqueous fermentation medium comprises a total of glucose, sucrose, fructose, maltose and maltotriose in a concentration of at least 3 g, per 100 mL aqueous fermentation medium,, preferably at least 4 g, more preferably at least 5 g, even more preferably at least 6 g, even more preferably at least 7 g of sugars selected from glucose, sucrose, fructose, maltose and maltotriose, per 100 mL aqueous fermentation medium.

Preferably, said aqueous fermentation medium comprises an original gravity at least 1.004 (1 °P), more preferably at least 1.016 (or 4 °P), preferably at least 1.020 (or 5 °P), more preferably at least 1.024 (or 6 °P). Preferably, said aqueous fermentation medium comprises an original gravity of between about 1.016 (or 4 °P) and about 1.120 (or 30 °P), more preferably between about 1.016 (or 4 °P) and about 1080 (or 20 °P), even more preferably between about 1020 (or 5 °P) and about 1048 (or 12 °P), most preferably between about 1020 (or 5 °P) and 1048 (or 8 °P), most preferably between about 1020 (or °P) and about 1024 (or 6 °P).

Said aqueous fermentation medium preferably comprises, per 100 mL aqueous fermentation medium, at least 0.8 g of a total of maltose and maltotriose, preferably at least 1.0 g, more preferably at least 1.2 g, more preferably at least 1.5 g, even more preferably at least 2 g, even more preferably at least 2.5 g, most preferably at least 3 g, per 100 mL aqueous fermentation medium,.

Preferably, the w/w ratio between maltose and maltotriose is between about 5:1 and about 2:1, more preferably between about 4:1 and 3:1.

Preferably said aqueous fermentation medium comprises maltose in an amount of at least 0.8 g. More preferably said aqueous fermentation medium comprises at least 0.9 g, more preferably at least 1 g, even more preferably 1.5 g, even more preferably at least 2 g, even more preferably at least 2.5 g of maltose, per 100 mL aqueous fermentation medium. Preferably, said aqueous fermentation medium comprises maltose in an amount of between about 1 g and about 6 g, preferably between about 1.5 g and about 5 g, more preferably between about 2 g and about 4 g, in particular between about 2.5 g and about 3.5 g, per 100 mL aqueous fermentation medium.

Preferably, said aqueous fermentation medium comprises maltotriose in an amount of at least 0.2 g per 100 mL aqueous fermentation medium. More preferably said aqueous fermentation medium comprises at least 0.3 g, even more preferably at least 0.5 g, even more preferably at least 0.7 g of maltotriose, per 100 mL aqueous fermentation medium,. Preferably, said aqueous fermentation medium comprises maltotriose in an amount of between about 0.2 g and about 2 wg, preferably between about 0.3 g and about 1.5 g, more preferably between about 0.4 g and about 1 g, per 100 mL aqueous fermentation medium.

Preferably, the total of sucrose, fructose and glucose is at most 0.8 g, more preferably at most 0.7 g, even more preferably at most 0.5 g, most preferably at most 0.3 g, per 100 mL aqueous fermentation medium,. Preferably, the total of glucose, fructose and sucrose is at least 0.1 g, more preferably at least 0.2 g, even more preferably at least 0.5 g, per 100 mL aqueous fermentation medium. Preferably, said aqueous fermentation medium comprises between about 0 g and about 0.8 g of a total of fructose, glucose and sucrose, more preferably between about 0.1 g and about 0.7 g of fructose, glucose and sucrose, even more preferably between about 0.2 g and about 0.5 of a total of glucose, fructose and sucrose, per 100 mL aqueous fermentation medium.

Said aqueous fermentation medium further preferably comprises, per 100 mL, glucose in an amount of at least 0.2 g. More preferably said aqueous fermentation medium comprises at least 0.3 g, even more preferably at least 0.4 g, even more preferably at least 0.5 g of glucose, even more preferably at least 0.6 g, most preferably at least 0.7 g of glucose, per 100 mL aqueous fermentation medium. Preferably, said aqueous fermentation medium comprises, per 100 mL, at most 5 g of glucose, more preferably at most 4 g of glucose, even more preferably at most 3 g of glucose, in particular at most 2 g of glucose, more in particular at most 1.5 g of glucose, more in particular at most 1 g of glucose, even more in particular at most 0.6 g, even more in particular at most 0.5 g of glucose, even more in particular at most 0.3 g, in particular is essentially free of glucose. Preferably, said aqueous fermentation medium comprises glucose in an amount of between about 0 g and about 5 g, preferably between about 0.1 g and about 3 g, more preferably between about 0.2 g and about 1.5 g, even more preferably between about 0.3 g and about 0.6 g, per 100 mL aqueous fermentation medium.

Said aqueous fermentation medium further preferably comprises, per 100 mL, fructose in an amount of at least 0.05 g. More preferably said aqueous fermentation medium comprises at least 0.08 g, even more preferably at least 0.1 g, even more preferably at least 0.12 g of fructose, per 100 mL aqueous fermentation medium. Preferably, said aqueous fermentation medium comprises, per 100 mL, at most 1 g of fructose, more preferably at most 0.5 g of fructose, even more preferably at most 0.4 g of fructose, in particular at most 0.3 g of fructose, more in particular at most 0.2 g of fructose, even more in particular at most 0.1 g of fructose in particular is essentially free of fructose. Preferably, said aqueous fermentation medium comprises, per 100 mL, fructose in an amount of between about 0 g and about 1 g, preferably between about 0.05 g and about 0.5 g, more preferably between about 0.08 g and about 0.12 g, , per 100 mL aqueous fermentation medium.

Said aqueous fermentation medium further preferably comprises, per 100 mL, sucrose in an amount of at least 0.05 g. More preferably said aqueous fermentation medium comprises at least 0.08 g, even more preferably at least 0.1 g, even more preferably at least 0.12 g of sucrose, per 100 mL aqueous fermentation medium. Preferably, said aqueous fermentation medium comprises, per 100 mL, at most 1 g of sucrose, more preferably at most 0.8 g of sucrose, even more preferably at most 0.5 g of sucrose, in particular at most 0.2 g of sucrose, more in particular at most 0.1 g of sucrose, in particular is essentially free of sucrose. Preferably, said aqueous fermentation medium comprises, per 100 mL, sucrose in an amount of between about 0 g and about 1 g, preferably between about 0.05 g and about 0.5 g, more preferably between about 0.08 g and about 0.12 g, per 100 mL aqueous fermentation medium.

Typically, said aqueous fermentation medium comprises non-fermentable sugars, preferably dextrins. Usually, said aqueous fermentation medium comprises, per 100 mL aqueous fermentation medium, at least 0.1 g of non-fermentable sugars, preferably dextrins, more preferably at least 0.5 g of non-fermentable sugars, preferably dextrins, even more preferably at least 0.9 g of non-fermentable sugars, preferably dextrins, in particular at least 1.2 g of non-fermentable sugars, preferably dextrins. Preferably, said aqueous fermentation medium comprises, per 100 mL, between about 0.1 g and about 3 g of non-fermentable sugars, preferably dextrins, more preferably between about 0.5 g and about 2 g of non-fermentable sugars, preferably dextrins.

Said aqueous fermentation further optionally comprises one or more components selected from amino acids, peptides, proteins, lipids, hop bitters, hop oils, and salts.

Said aqueous fermentation medium further comprises a plurality of yeast cells. Preferably, said aqueous fermentation medium, at the start of said fermentation, comprises a plurality of yeast cells in an amount of at least about 1 ·10⁶ cells/ml, more preferably at least about 2 ·10⁶ cells/ml, more preferably at least about 3 ·10⁶ cells/ml. Preferably, said aqueous fermentation medium comprises a plurality of yeast cells of between about 1 ·10⁶ cells/ml and about 1 ·10⁷ cells/ml, more preferably between about 1 ·10⁶ cells/ml and about 5 ·10⁶ cells/ml, even more preferably between about 1 ·10⁶ cells/ml and about 4 ·10⁶ cells/ml, such as around 3 ·10⁶ cells/ml.

Said aqueous fermentation usually has a pH of between about 5 and about 6, in particular about 5.5. Optionally the pH of said aqueous fermentation medium is reduced with about 0.4 pH units or more. The pH of said aqueous fermentation medium may be reduced at any suitable time during the method according to the invention. For example, the pH may be reduced before or after culturing of the aqueous fermentation medium. Preferably, said pH is reduced prior to culturing said aqueous fermentation medium. Preferably said pH is reduced with 0.4 pH units or more, more preferably with 0.5 pH units or more, even more preferably with 0.6 pH units or more, in particular with 0.8 pH units or more. Such pH values are suitable for obtaining a microbially stable fermented beverage.

Alternatively or additionally, the pH of said aqueous fermentation medium, prior to culturing is preferably set to at most 5.3, preferably at most 5, more preferably at most 4.8, even more preferably at most 4.6. Preferably, the pH is set to between about 4.6 and about 5. Alternatively or additionally, the pH of said aqueous fermentation medium, after said culturing, is preferably set to a pH of 4.5 or less, preferably 4.4 or less, more preferably 4.3 or less, even more preferably 4.2 or less. Preferably, the pH of said aqueous fermentation medium, after said culturing, is set to a pH of at most 3.8, more preferably at most 3.9, even more preferably at most 4. Preferably, the pH is set to between about 3.8 and about 4.5, more preferably between about 4 and about 4.3. Such pH values provide a microbially stable fermented beverage. Such pH values provide a microbially stable fermented beer beverage with good taste.

Said aqueous fermentation medium may be set to the defined pH using a suitable acidulant. Examples include but are not limited to acetic acid, citric acid, malic acid, lactic acid, tartaric acid and phosphoric acid.

Said aqueous fermentation medium is subsequently cultured under conditions allowing the yeast to ferment the aqueous fermentation medium to obtain a fermented beverage.

Said aqueous fermentation medium is preferably cultured at a temperature of at least 5 °C, more preferably at least 10 °C, more preferably at least 16 °C, even more preferably at least 20 °C. Preferably said aqueous fermentation medium is cultured at a temperature of between about 5 °C and about 30 °C, more preferably between about 10 °C and about 25 °C, such as between about 16 and about 22 °C.

Said aqueous fermentation medium is cultured under conditions allowing the yeast to convert one or more of 2-methylpropanal, 2-methylbutanal, 3-methylbutanal, methional, E-2-nonenal, 4-vinyl guaiacol, diacetyl and hexanal, preferably hexanal, present in the wort to a content below the flavor threshold and/or to convert one or more sucrose, fructose and glucose, thereby producing at most 0.5 vol.% of alcohol, preferably at most 0.2 vol.% of alcohol, based on the total volume of the aqueous fermentation medium.

Said aqueous fermentation medium is preferably cultured for a time sufficient to allow decrease of the pH of said aqueous fermentation medium with 0.5 units of pH or more, preferably 0.6 units of pH or more, more preferably 0.7 units of pH or more, preferably at most 0.8 units of pH or more, even more preferably 0.9 units of pH or more.

Usually, said aqueous fermentation medium is cultured for at least 12 h, more preferably at least 16 h, most preferably at least 20 h. Typically, said aqueous fermentation medium is cultured for at most 120 h, preferably at most 96 h, even more preferably at most 48 h. Preferably, said aqueous fermentation medium is cultured for between about 12 h and about 120 h, more preferably between about 16 h and about 96 h, most preferably between about 18 h and about 72 h, such as between about 20 h and about 30 h.

The inventors surprisingly realized that with a method according to the invention, the time required to convert glucose, fructose and sucrose, if present, into alcohol is relatively short compared to other commercially available yeast used in production of fermented beverages, in particular beer.

Accordingly, the invention further relates to a method according to the invention wherein at least 15 wt.% of a total of glucose, fructose and sucrose is converted in a time of 8 h or less, preferably 6 h or less and/or wherein at least 80 wt.%, preferably at least 85 wt.%, more preferably at least 90 wt.%, even more preferably at least 92 wt.%, even more preferably at least 95 wt.%, in particular at least 99 wt.% of said total of glucose, fructose and sucrose is converted in a time of 16 h or less, preferably 12 h or less.

The cultured aqueous fermentation medium obtained in step c) may optionally be diluted to obtain an aqueous fermentation medium comprising at most 0.5 vol.% alcohol. This usually depends on the gravity of the wort that is used for fermentation, wherein a higher gravity wort usually produce a higher content of alcohol compared to lower gravity wort. It is an advantage of the method of the invention that a fermented aqueous fermentation medium may be obtained with relatively low alcohol content, especially relatively low alcohol content compared to a regular yeast for fermentation of wort. Thereby, a lower degree of dilution is required to obtain an aqueous fermentation medium comprising at most 0.5 vol.% alcohol, thereby preventing excessive dilution of flavor components and allowing to obtain an aqueous fermentation medium with relatively high content of flavor components. Such ana aqueous fermentation medium is usually perceived as less watery and stronger body.

Optionally, said fermented beverage may be isolated from said cultured fermentation medium. Preferably, said fermented beverage is subjected to a separation step to remove solid particles from said fermented beverage. More preferably said separation step comprises one or more filtration steps and/or centrifugation steps.

Optionally the fermented beverage may be subjected to one or more processing steps such as maturation or filtration. The fermented beverage may optionally be packaged in a can, bottle, or barrel.

The invention further relates to a use of a yeast cell or a yeast preparation according to the invention to prepare a fermented beverage.

### Fermented beverage

During culturing said yeast converts worty off-flavors present in the wort to a level below the flavor threshold, to produce a fermented beverage with acceptable taste. Typical components imparting off-flavors are aldehydes formed by Strecker-degradation of amino acids (also referred to as Strecker aldehydes). Examples include 2-methylbutanal, 2-methylpropanol, 3-methylbutanal, methional, and phenylacetaldehyde. Other aldehydes that may impart off taste include hexanal, trans-2-nonenal, 4-vinyl guaiacol and 5-hydroxy-methyl-furfural.

Accordingly, the invention further relates to a fermented beverage, preferably obtainable by a method according to the invention, which fermented beverage comprises ethanol in a concentration of 0.5 vol% or less, based on the total volume of the fermented beverage, and one or more of 2-methylpropanal in a concentration of 80 ppb or less, preferably 20 ppb or less, 2-methylbutanal in a concentration of 40 ppb or less, preferably 10 ppb or less, 3-methylbutanal in a concentration of 50 ppb or less, preferably 20 ppb or less, hexanal in a concentration of 80 ppb or less, preferably 40 ppb or less, methional in a concentration of 4 ppb or less, E-2-nonenal in a concentration of 0.1 ppb or less, preferably 0.03 ppb or less, 4-vinyl-guaiacol in a concentration of 200 ppb or less, preferably 100 ppb or less, most preferably 50 ppb or less and diacetyl in a concentration of 50 ppb or less, preferably 20 ppb or less, based on the total volume of fermented beverage.

Preferably the fermented beverage is a beer, more preferably a low-alcoholic beer or an alcohol-free beer. Preferably, the beer has an alcohol content of at most 1.2 vol.%, more preferably at most 1.0 vol.%, even more preferably at most 0.8 vol.%, even more preferably at most 0.5 vol.%, even more preferably at most 0.3 vol.%, even more preferably at most 0.2 vol.%, even more preferably at most 0.1 vol.%, even more preferably at most 0.2 vol.%, most preferably essentially free of alcohol.

The invention preferably relates to a fermented beverage, wherein the contents of one or more of, preferably two or more, more preferably three or more, even more preferably four or more, even more preferably five or more, even more preferably six or more, even more preferably seven or more, most preferably eight components selected from 2-methylbutanal, 2-methylpropanol, 3-methylbutanal, hexanal, methional, phenylacetaldehyde, 4-vinyl guaiacol and trans-2-nonanal are below the flavor threshold, more preferably wherein methional is below the flavor threshold. The flavor threshold is as described by Gernat et al. 2020. Food and Bioprocess Technology 13:195-216.

Preferably, the content of 2-methylpropional in a fermented beverage according to the invention is 80 ppb or less, more preferably 60 ppb or less, most preferably 40 ppb or less. Preferably, the content of 2-methylpropional is in the range of about 1 ppb and about 80 ppb, more preferably in the range of about 2 ppb and about 60 ppb, such as in the range of about 3 ppb and about 20 ppb.

Preferably, the content of 2-methylbutanal in a fermented beverage according to the invention is 40 ppb or less, more preferably 20 ppb or less, most preferably 10 ppb or less. Preferably, the content of 2-methylbutanal is in the range of about 1 ppb and about 40 ppb, more preferably in the range of about 2 ppb and about 20 ppb, such as in the range of about 20 ppb and about 10 ppb.

Preferably, the content of 3-methylbutanal in a fermented beverage according to the invention is 50 ppb or less, more preferably 30 ppb or less, most preferably 20 ppb or less, even more preferably 10 ppb or less. Preferably, the content of 3-methylbutanal is in the range of about 1 ppb and about 50 ppb, more preferably in the range of about 3 ppb and about 30 ppb, such as in the range of about 5 ppb and about 10 ppb.

Preferably, the content of methional in a fermented beverage according to the invention is 50 ppb or less, more preferably 20 ppb or less, even more preferably 5 ppb or less, even more preferably 4 ppb or less, even more preferably 3 ppb or less, most preferably 2.5 ppb or less. Preferably, the content of methional is in the range of about 1 ppb and about 5 ppb, more preferably in the range of about 2 ppb and about 4.2 ppb, such as in the range of about 2 ppb and about 2.5 ppb.

Preferably, the content of hexanal in a fermented beverage according to the invention is 80 ppb or less, more preferably 50 ppb or less, most preferably 20 ppb or less. Preferably, the content of hexanal is in the range of about 0.1 ppb and about 80 ppb, more preferably in the range of about 0.3 ppb and about 50 ppb, such as in the range of about 0.5 ppb and about 20 ppb.

Preferably, the content of E-2-nonenal in a fermented beverage according to the invention is 0.1 ppb or less, more preferably 0.05 ppb or less, most preferably 0.02 ppb or less. Preferably, the content of E-2-Nonenal is in the range of about 0.001 ppb and about 0.1 ppb, more preferably in the range of about 0.005 ppb and about 0.1 ppb, such as in the range of about 0.01 ppb and about 0.05 ppb.

Preferably, the content of diacetyl is 50 ppb or less, more preferably 30 ppb or less, most preferably 20 ppb or less. Preferably, the content of diacetyl is in the range of about 1 ppb and 50 ppb, more preferably 1 ppb and 30 ppb, most preferably 1 ppb and 20 ppb.

Preferably, the content of 4-vinyl guaiacol is 1200 ppb or less, preferably 1000 ppb or less, more preferably 500 ppb or less, even more preferably 200 ppb or less, even more preferably 150 ppb or less, even more preferably 120 ppb or less, in particular 100 ppb or less.

During culturing said yeast converts flavor precursors, in particular originating from added hops or dry-hopping, to flavor molecules.

The inventors found that a fermented beverage according to the invention, preferably a (low alcohol) beer has a relatively hoppy flavor, especially relatively hoppy flavor compared to a low alcohol beer fermented with commercially available yeast cells for preparing low alcohol beer. It was further found that a fermented beverage according to the invention has relatively fruity flavor, especially relatively fruity compared to low alcohol beer fermented with commercially available yeast cells for preparing low alcohol beer. It was further found that the fermented beverage according to the invention, preferably a (low alcohol) beer has relatively low sweetness, especially relatively low sweetness compared to low alcohol beer fermented with commercially available yeast cells for preparing low alcohol beer. It was further found that the fermented beverage according to the invention, preferably a (low alcohol) beer has relatively low worthy/grainy flavor, especially relatively low worthy/grainy compared to low alcohol beer fermented with commercially available yeast cells for preparing low alcohol beer.

Preferably, said fermented beverage has a content of ethyl acetate of at least 3000 ppb, more preferably at least 3500 ppb, even more preferably at least 3800 ppb, most preferably at least 4000 ppb. Preferably said fermented beverage has a content of ethyl acetate in the range of between about 3000 ppb and about 5000 ppb, preferably between about 3500 ppb and about 4500 ppb, in particular between about 3800 ppb and about 4200 ppb.

Preferably, said fermented beverage has a content of 2-phenylethyl acetate of at least 3800 ppb, more preferably at least 4000 ppb, even more preferably at least 4500 ppb, most preferably at least 5000 ppb. Preferably said fermented beverage has a content of 2-phenylacetate in the range of between about 3800 ppb and about 8000 ppb, preferably between about 4000 ppb and about 6000 ppb, in particular between about 4500 ppb and about 5000 ppb.

Preferably, said fermented beverage has a content of ethyl caproate of at least 210 ppb, more preferably at least 250 ppb, even more preferably at least 300 ppb, most preferably at least 400 ppb. Preferably said fermented beverage has a content of ethyl caproate in the range of between about 210 ppb and about 2000 ppb, preferably between about 250 ppb and about 1000 ppb, in particular between about 300 ppb and about 800 ppb.

Preferably, said fermented beverage has a content of ethyl caprylate of at least 900 ppb, more preferably at least 100 ppb, even more preferably at least 1500 ppb, most preferably at least 2000 ppb. Preferably said fermented beverage has a content of ethyl caprylate in the range of between about 900 ppb and about 5000 ppb, preferably between about 1000 ppb and about 3000 ppb, in particular between about 1500 ppb and about 2000 ppb.

Preferably, said fermented beverage has a content of linalool of at least 5 ppb, more preferably at least 10 ppb, even more preferably at least 20 ppb, even more preferably at least 80 ppb, even more preferably at least 150 ppb, even more preferably at least 300 ppb, most preferably at least 500 ppb. Preferably, said fermented beverage has a content of linalool in the range of between about 5 ppb and about 5000 ppb, even more preferably between about 10 ppb and about 2000 ppb, even more preferably between about 20 ppb and about 1000 ppb.

Preferably, said fermented beverage has a content of geraniol of at least 5 ppb, more preferably at least 10 ppb, even more preferably at least 20 ppb, even more preferably at least 80 ppb, even more preferably at least 150 ppb, even more preferably at least 250 ppb, most preferably at least 500 ppb. Preferably, said fermented beverage has a content of geraniol in the range of between about 5 ppb and about 5000 ppb, even more preferably between about 50 ppb and about 2000 ppb, even more preferably between about 250 ppb and about 1000 ppb.

Preferably, said fermented beverage has a content of geranyl acetate of at least 50 ppb, more preferably at least 100 ppb, even more preferably at least 200 ppb, even more preferably at least 500 ppb, even more preferably at least 800 ppb, even more preferably at least 1000 ppb, most preferably at least 5000 ppb. Preferably, said fermented beverage has a content of geraniol in the range of between about 50 ppb and about 20000 ppb, even more preferably between about 100 ppb and about 10000 ppb, even more preferably between about 500 ppb and about 5000 ppb.

Preferably, said fermented beverage has a content of β-damascenone of at least 1 ppb, more preferably at least 5 ppb, even more preferably at least 30 ppb, even more preferably at least 80 ppb, even more preferably at least 150 ppb, even more preferably at least 300 ppb, most preferably at least 500 ppb. Preferably, said fermented beverage has a content of β-damascenone in the range of between about 1 ppb and about 5000 ppb, even more preferably between about 5 ppb and about 2000 ppb, even more preferably between about 150 ppb and about 1000 ppb.

Preferably, said fermented beverage has a content of β-ionone of at least 1 ppb, more preferably at least 5 ppb, even more preferably at least 10 ppb, even more preferably at least 50 ppb, even more preferably at least 150 ppb, even more preferably at least 300 ppb, most preferably at least 500 ppb. Preferably, said fermented beverage has a content of β-ionone in the range of between about 1 ppb and about 5000 ppb, even more preferably between about 5 ppb and about 2000 ppb, even more preferably between about 10 ppb and about 1000 ppb.

Preferably, said fermented beverage has a content of β-myrcene of at least 1 ppb, more preferably at least 5 ppb, even more preferably at least 10 ppb, even more preferably at least 50 ppb, even more preferably at least 150 ppb, even more preferably at least 300 ppb, most preferably at least 500 ppb. Preferably, said fermented beverage has a content of β-myrcene in the range of between about 1 ppb and about 5000 ppb, even more preferably between about 50 ppb and about 2000 ppb, even more preferably between about 100 ppb and about 1000 ppb.

Preferably, said fermented beverage has a content of humulenol of at least 20 ppb, more preferably at least 50 ppb, even more preferably at least 100 ppb, even more preferably at least 200 ppb, even more preferably at least 500 ppb, even more preferably at least 1000 ppb, most preferably at least 2000 ppb. Preferably, said fermented beverage has a content of humulenol in the range of between about 20 ppb and about 5000 ppb, even more preferably between about 50 ppb and about 2000 ppb, even more preferably between about 500 ppb and about 1000 ppb.

Preferably, said fermented beverage has a content of α-humulene of at least 20 ppb, more preferably at least 50 ppb, even more preferably at least 100 ppb, even more preferably at least 350 ppb, even more preferably at least 500 ppb, even more preferably at least 1000 ppb, most preferably at least 2000 ppb. Preferably, said fermented beverage has a content of α-humulene in the range of between about 50 ppb and about 5000 ppb, even more preferably between about 350 ppb and about 2000 ppb, even more preferably between about 500 ppb and about 1000 ppb.

Preferably, said fermented beverage has a content of nerol of at least 20 ppb, more preferably at least 50 ppb, even more preferably at least 100 ppb, even more preferably at least 500 ppb, even more preferably at least 1000 ppb, even more preferably at least 2000 ppb, most preferably at least 3000 ppb. Preferably, said fermented beverage has a content of nerol in the range of between about 20 ppb and about 20000 ppb, even more preferably between about 350 ppb and about 10000 ppb, even more preferably between about 800 ppb and about 5000 ppb.

Preferably, said fermented beverage has a content of β-carophyllene of at least 200 ppb, more preferably at least 500 ppb, even more preferably at least 700 ppb, even more preferably at least 800 ppb, even more preferably at least 1000 ppb, even more preferably at least 2000 ppb, most preferably at least 3000 ppb. Preferably, said fermented beverage has a content of β-carophyllene in the range of between about 50 ppb and about 20000 ppb, even more preferably between about 350 ppb and about 10000 ppb, even more preferably between about 800 ppb and about 5000 ppb.

Preferably, said fermented beverage has a content of β-citronellol of at least 5 ppb, more preferably at least 10 ppb, even more preferably at least 40 ppb, even more preferably at least 50 ppb, even more preferably at least 100 ppb, even more preferably at least 500 ppb, most preferably at least 1000 ppb. Preferably, said fermented beverage has a content of β-citronellol in the range of between about 5 ppb and about 20000 ppb, even more preferably between about 40 ppb and about 10000 ppb, even more preferably between about 100 ppb and about 5000 ppb.

Preferably, said fermented beverage has a content of farnesene of at least 5 ppb, more preferably at least 10 ppb, even more preferably at least 20 ppb, even more preferably at least 80 ppb, even more preferably at least 150 ppb, even more preferably at least 300 ppb, most preferably at least 500 ppb. Preferably, said fermented beverage has a content of farnesene in the range of between about 5 ppb and about 5000 ppb, even more preferably between about 10 ppb and about 2000 ppb, even more preferably between about 20 ppb and about 1000 ppb.

Preferably, said fermented beverage has a content of 4-mercapto-4-methylpentant-2-one (4MMP) of at least 0.001 ppb, more preferably at least 0.004 ppb, even more preferably at least 0.01 ppb. Preferably, said fermented beverage has a content of 4MMP in the range of between about 0.00001 ppb and about 100 ppb, even more preferably between about 0.001 ppb and about 10 ppb, even more preferably between about 0.01 ppb and about 1 ppb.

Preferably, said fermented beverage has a content of 3-mercapto-4-methylpentan-1-ol (3M4MP) of at least 0.01 ppb, more preferably at least 0.07 ppb, even more preferably at least 0. 1 ppb, even more preferably at least 1 ppb. Preferably, said fermented beverage has a content of 3M4MP in the range of between about 0.001 ppb and about 100 ppb, even more preferably between about 0.07 ppb and about 10 ppb, even more preferably between about 0.1 ppb and about 1 ppb.

Preferably, said fermented beverage has a content of 3-sulfanyl-1-hexanol (3MH) of at least 0.01 ppb, more preferably at least 0.06 ppb, even more preferably at least 0.1 ppb, even more preferably at least 1 ppb. Preferably, said fermented beverage has a content of 3-sulfanyl-1-hexanol in the range of between about 0.001 ppb and about 100 ppb, even more preferably between about 0.06 ppb and about 10 ppb, even more preferably between about 0.1 ppb and about 1 ppb.

Preferably, said fermented beverage has a content of 3-mercaptohexyl acetate (3MHA) of at least 0.001 ppb, more preferably at least 0.004 ppb, even more preferably at least 0.01 ppb, even more preferably at least 0.1 ppb. Preferably, said fermented beverage has a content of 3MHA in the range of between about 0.00001 ppb and about 100 ppb, even more preferably between about 0.001 ppb and about 10 ppb, even more preferably between about 0.01 ppb and about 1 ppb.

For the purpose of clarity and a concise description, features are described herein as part of the same or separate embodiments, however, it will be appreciated that the scope of the invention may include embodiments having combinations of all or some of the features described.

The invention is demonstrated by the following examples.

### Examples

### Example 1: fermentation of an aqueous fermentation medium with a yeast according to the invention

300 mL of an aqueous fermentation medium was provided consisting of dry malt extract-based wort and a plurality of yeast cells as deposited with the National Measurement Institute (Victoria, Australia) under accession number V24/019646 in an amount of 3.5 · 10⁶.

The pH of the fermentation medium was set to 4.75 using phosphoric acid.

The aqueous fermentation medium was charged into a fermentation vessel, which was sealed and equipped with an automated CO₂ release valve and monitoring system to prevent oxygen from entering the fermentation medium and detect CO₂ formed during the experiment. The aqueous fermentation medium was allowed to stand at 20 °C and was stirred at 100 rotations per minute with a magnetic stirring rod.

The aqueous medium contains 2.4 g maltose, 0.58 g glucose, 0.70 g maltotriose, 0.09 g sucrose, 0.1 g of fructose and 0.9 g of dextrins, per 100 mL aqueous fermentation medium.

The sugar content of fermentation media was determined by analysis with High Pressure liquid Chromatography (HPLC). Samples were prepared by transferring 35 ml of ferment to a 50 ml Greiner tube and centrifuging with a Sorvall Legend X1R centrifuge (Thermo Fisher Scientific, Landsmeer, The Netherlands) at 4 °C and 5000 rpm for 10 minutes. Then, 2 ml of supernatant was transferred to a 1.5 ml HPLC vial via a syringe with a 0.2 µm syringe filter (Thermo Fisher Scientific, Landsmeer, The Netherlands). The vials were stored at -20 °C until analysis. HPLC samples were injected at an injection size of 5 µl into an Agilent (Amstelveen, The Netherlands) 1260 infinity HPLC system containing a BioRad HPX-87H (300 x 7.8 mm) column (Bio-Rad Laboratories GmbH, Feldkirchen, Germany), kept at 60 °C, and a refractive index detector (RID), at 40 °C. A 5 mM H₂SO₄ solution was used as eluent at a flowrate of 0.6 ml/min for a total runtime of 30 minutes. A premade mixture of compounds (AB Mauri, Sydney, Australia) including maltodextrin, maltotriose, maltose, glucose, fructose, lactic acid, glycerol, acetic acid, and ethanol was used as a calibration standard.

After 72 h, a sample was taken from the aqueous fermentation medium and the pH was determined using a calibrated pH meter. The results are shown in Table 1. The experiment was repeated using Lallemand LoNa^{®} (commercially available from Lallemand Brewing) and SafAle LA-01 (commercially available from Fermentis).

**Table 1- pH values and 4-vinyl guaiacol levels of fermentation medium fermented with V24/019646 compared to LoNa^{®} and SafAle LA01; n.d. = not detected.**

| Yeast | pH | 4-vinyl guaiacol (g/mL) |
|---|---|---|
| V24/019646 | 3.79 +/- 0.02 | n.d. |
| LoNa^{®} | 4.10 +/- 0.16 | n.d. |
| SafAle LA-01 | 4.15 +/- 0.01 | 1.2 |

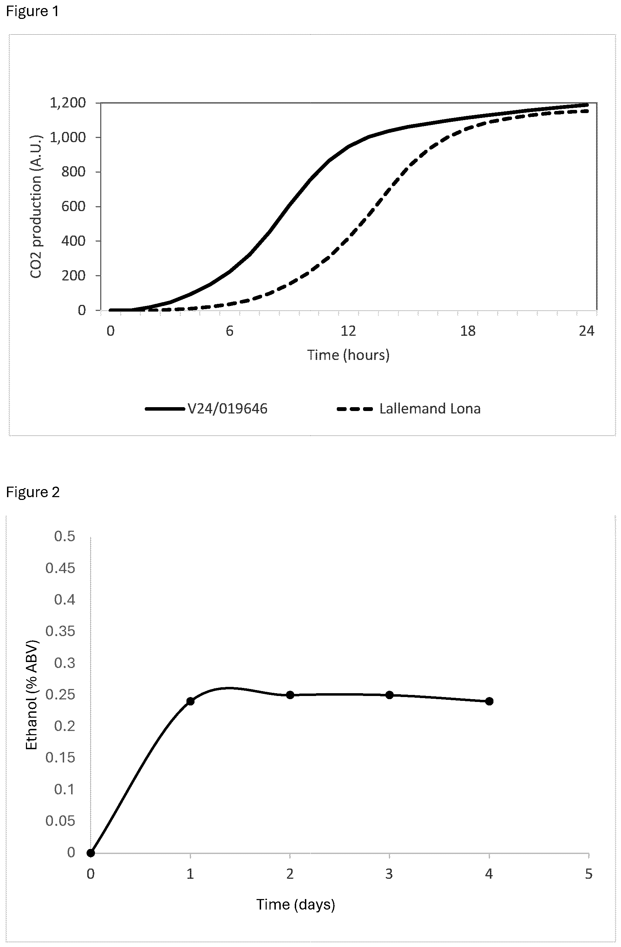

Simultaneously, the cumulative CO₂ pressure was monitored over time. The results are shown in Figure 1. As a reference, Lallemand LoNa^{®} was included.

It was further found that the yeast cell according to the invention is capable of reducing the pH to a stronger degree than Lallemand LoNa^{®} and SafAle LA-01 (Table 1). Moreover, the results show that the yeast according to the invention had a faster fermentation time compared to Lallemand LoNa^{®} (Figure 1).

### Example 2: Determining off-flavor molecules of a fermented beverage according to the invention

A wort of 12°P was made with 100% malt, with a regular pilsner-type mashing scheme and acidified after boiling to pH 5. Bitter hops were added at start of boiling up to 20 IBU. The wort was diluted in 4 fermentation tanks with sterilized brewing water to 5.5°P. Yeast was added and 275 g/hl of citra hop was added at the start of fermentation. V24/019646, Lallemand Lona and SafAle LA01 were added at 50 g/hl each in tank 1, 2 and 3. A control without dry hopping was done with V24/019646 in a 4th tank. The fermentation was carried out for 4 days at 20°C. Thereafter the tank was cooled to 0°C. The beer was matured during 10 days. Aldehyde content was determined after 0, 1, 2, 3 and 4 days of fermentation and after 10 days of maturation. The results are shown in tables 2 and 3.

Further, the alcohol content was determined over time by NIR (Anton Paar Alcoholyzer). The results are shown in Figure 2. As can be derived from Figure 2, the total alcohol content is below 0.5 vol.%.

It was found that a yeast cell according to the invention was capable of fermenting an aqueous fermentation medium comprising, per 100 mL, 2.4 g of maltose and 0.7 g of maltotriose, whilst producing less than 0.5 vol.% of alcohol (Figure 2). Hence, the yeast is suitable for preparation of low alcoholic beverages.

The content of off-flavor components was determined in the fermented beverages as obtained in example 1 using the method as described in Dennenlöhr, J., Thörner, S., Maxminer, J., & Rettberg, N. (2020). Analysis of Selected Staling Aldehydes in Wort and Beer by GC-EI-MS/MS Using HS-SPME with On-Fiber Derivatization. Journal of the American Society of Brewing Chemists, 78(4), 284-298. The results are shown in Table 2 and compared to the flavour threshold as described by Gernat et al. 2020. Food and Bioprocess Technology 13:195-216.

**Table 2 - Aldehyde levels in a V24/019646 fermentation over time (ppb)**

| Aldehyde | 2-MP | 2-MB | 3-MP | Hexanal | Furfural | Methional | Phenyl-acetaldehyde | Trans-2-nonenal |
|---|---|---|---|---|---|---|---|---|
| Flavour threshold | 86-1000 | 45-1250 | 56-600 | 88-350 | 15000-150000 | 4.2-250 | 105-1600 | 0.03-0.11 |
| 0 (starting wort) | 17.7 | 10.4 | 33.1 | 3.49 | 246 | 48.6 | 47.1 | 0.11 |
| Day 1 | 10.7 | 5.41 | 18.5 | 1.06 | 2.09 | 3.17 | 18 | 0.03 |
| Day 2 | 10.7 | 5.37 | 15.5 | 0.94 | 1.78 | 1.96 | 14 | 0.02 |
| Day 3 | 6.07 | 3.2 | 9.46 | 0.91 | 1.87 | 2.08 | 6.43 | 0.02 |
| Day 4 | 3.96 | 2.16 | 5.04 | 90.93 | 1.54 | 2.05 | 4.72 | 0.02 |
| 10d maturation | 3.41 | 2.20 | 5.36 | 0.70 | 1.45 | 3.02 | 7.00 | 0.02 |

### Example 3: Sensory evaluation of a fermented beverage according to the invention

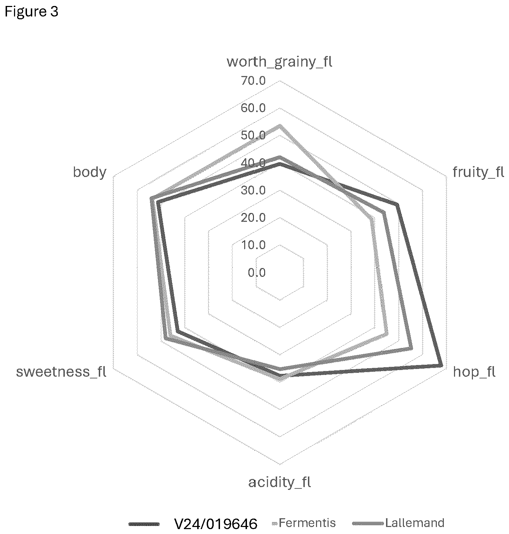

A sensory evaluation by a trained panel was performed on the fermented beverage as obtained in Example 2 and compared to a fermented beverage obtained with example 2 when using yeast cells of Lallemand LoNa^{®} and Fermentis SafAle LA01. The results are shown in Table 3 and Figure 3.

**Table 3 - Average perceived intensity of the evaluated attributes; Different superscripts in the same column attribute indicate significant differences between samples.**

| Strain | Worth/grains | Fruity | Hop | Acidity | Sweetness | Body |
|---|---|---|---|---|---|---|
| V24/019646 | 39.6 | 49.3 | 67.9^{a} | 37.7 | 42.9 | 51.3 |
| SafAle LA01 | 53.5 | 38.7 | 44.9^{b} | 38.9 | 46.0 | 53.7 |
| Lallemand LoNa | 42.1 | 43.7 | 55.3^{b} | 35.3 | 48.0 | 54.0 |

It was found that the fermented beverage obtained by fermentation of an aqueous fermentation medium using a yeast according to the invention had statistically significant more hoppy notes, compared to a fermented beverage prepared with Lallemand LoNa^{®} and Fermentis SafAle LA01.

## Claims

1. A yeast cell that is capable of fermenting an aqueous fermentation medium comprising, per 100 mL, at least 0.8 g of a total of maltose and maltotriose and a total of glucose, fructose and sucrose of at most 0.8 g, thereby producing alcohol in a concentration of 0.5 vol.% or less, based on the volume of the aqueous fermentation medium, and that has an ability to convert at least 15 wt.% of said total of glucose, fructose and sucrose in a time of 8 h or less and/or that has an ability to convert at least 80 wt.% of said total of glucose, fructose and sucrose in a time of 16 h or less.

2. A yeast cell, preferably according to claim 1, that is capable of fermenting an aqueous fermentation medium comprising, per 100 mL, at least 0.8 g of a total of maltose and maltotriose and a total of glucose, fructose and sucrose of at most 0.8 g, thereby producing alcohol in a concentration of 0.5 vol.% or less, based on the volume of the aqueous fermentation medium and that has an ability to decrease the pH of the aqueous fermentation medium by 0.7 pH units or more.

3. The yeast cell according to any of the preceding claims, wherein said ability to decrease the pH is determinable and/or said ability to convert at least 15 wt.% of said total of glucose, fructose and sucrose in a time of 8 h or less and/or that has an ability to convert at least 80 wt.% of said total of glucose, fructose and sucrose in a time of 16 h or less, by a method comprising the steps of:
- providing a fermentation medium consisting essentially of wort and a plurality of yeast cells in a concentration of 1 · 10⁶ cells/ml, said fermentation medium comprising, per 100 mL, 2.4 g maltose, 0.58 g glucose, 0.70 g maltotriose, 0.09 g sucrose, 0.1 g of fructose and 0.9 g of dextrins;
- setting the pH of said fermentation medium to 4.75;
- culturing the fermentation medium at a temperature of 20 °C for a time of 72 h in a sealed container configured to prevent oxygen from entering the container; and
- determining the pH of the cultured fermentation medium, wherein the yeast cell has an ability to decrease the pH of the aqueous fermentation medium or more if the pH of the cultured fermentation medium is 0.7 pH units lower than the pH of the aqueous fermentation medium prior to culturing; and/or
- measuring the CO₂ pressure over time and determining the amount of said total of glucose, sucrose and fructose based on the CO₂ pressure.

4. The yeast cell according to claim 3, wherein the wort is obtained by a process comprising the steps of:
- contacting crushed, germinated barley grains with water at a temperature of 63-72 °C for approximately 0.3-1.5 h, followed by increasing the temperature to 78 °C;
- separating the aqueous phase for the crushed germinated barley grains to obtain sweet wort;
- boiling the sweet wort to obtain boiled sweet wort; and
- adding hops or hop-derived products to the boiled sweet wort to obtain the wort.

5. The yeast cell according to any of the preceding claims, wherein the yeast cell is a yeast cell of the genus Saccharomyces, preferably a yeast cell of the species Saccharomyces cerevisiae.

6. A yeast cell, preferably according to any of the preceding claims, as deposited with the National Measurement Institute (Victoria, Australia) under accession number V24/019646 or a cell of a derivative strain thereof.

7. A yeast preparation, comprising a plurality of yeast cells according to any of the preceding claims, wherein said yeast preparation is a dry preparation, a liquid preparation, a cream preparation, a compressed preparation or a crumble preparation, preferably wherein said yeast preparation is a dry preparation.

8. A method for producing a fermented beverage, comprising
a) providing an aqueous fermentation medium comprising wort and a plurality of yeast cells according to any one of claims 1-6 or a yeast preparation according to claim 7,
b) optionally reducing the pH of said aqueous fermentation medium with 0.4 pH units or more;
c) culturing the aqueous fermentation medium under conditions allowing the yeast to convert one or more of 2-methylpropanal, 2-methylbutanal, 3-methylbutanal, methional, E-2-nonenal, 4-vinyl guaiacol, hexanal and optionally diacetyl, preferably hexanal, present in the wort to a content below the flavor threshold and/or to convert one or more of sucrose, fructose and glucose, thereby obtaining, optionally after dilution, an aqueous fermentation medium comprising at most 0.5 vol.% alcohol, based on the total volume of the aqueous fermentation medium; and
d) optionally isolating a fermented beverage from said aqueous fermentation medium.

9. The method according to claim 8, wherein the aqueous fermentation medium is cultured at a temperature of at least 5 °C, preferably at least 10 °C, more preferably at least 16 °C, most preferably at least 20 °C.

10. The method according to any of claims 8 or 9, wherein said aqueous fermentation medium comprises one or more of glucose, fructose and sucrose, and wherein at least 15 wt.% of a total of glucose, fructose and sucrose is converted in a time of 8 h or less and/or wherein at least 80 wt.% of said total of glucose, fructose and sucrose is converted in a time of 16 h or less.

11. The method according to any of claims 9 to 10, wherein said aqueous fermentation medium is cultured under conditions allowing a reduction in pH of at least 0.7 pH units or more.

12. The method according to any of claims 9 to 11, wherein the aqueous fermentation medium has an original gravity of at least 5 °P.

13. A fermented beverage, preferably obtainable by a method according to claim 8 to 12, which fermented beverage comprises ethanol in a concentration of 0.5 vol% or less, based on the total volume of the fermented beverage, and one or more of 2-methylpropanal in a concentration of 80 ppb or less, 2-methylbutanal in a concentration of 40 ppb or less, 3-methylbutanal in a concentration of 50 ppb or less, hexanal in a concentration of 80 ppb or less, methional in a concentration of 4 ppb or less, E-2-nonenal in a concentration of 0.02 ppb or less, 4-vinyl-guaiacol in a concentration of 200 ppb or less and optionally diacetyl in a concentration of 50 ppb or less, based on the total volume of fermented beverage.

14. The fermented beverage according to claim 13 which is a beer, preferably a low-alcoholic beer.

15. Use of a yeast cell according to any one of claims 1 to 6 or a yeast preparation according to claim 7 to prepare a fermented beverage.
